# EUROPEAN PATENT APPLICATION

(11) **EP 4 696 299 A1**
(43) Date of publication of application: **18.02.2026**
(21) Application number: 24788393.7
(22) Date of filing: 30.01.2024
(51) Int. Cl.: A61K 8/9728, A61K 8/34, A61K 36/06, A61P 17/16, A61Q 5/12, A61Q 19/00

(54) **TOPICAL COMPOSITION CONTAINING YEAST EXTRACT**

(30) Priority: 14.04.2023 JP 2023066240
(71) Applicant: Mitsubishi Corporation Life Sciences Limited, Tokyo 100-0006 (JP)
(72) Inventor: FUKUDA, Yusuke, Tokyo 100-0006 (JP); KATSUMATA, Tadayoshi, Tokyo 100-0006 (JP); TOMITAKA, Misa, Tokyo 100-0006 (JP); KOBAYASHI, Erina, Tokyo 100-0006 (JP); YOKOI, Yasuhito, Tokyo 100-0006 (JP)
(74) Representative: Müller-Boré & Partner Patentanwälte PartG mbB
(86) International application number: PCT/JP2024/002877
(87) International publication number: WO 2024/214363

(57) **Abstract**

[Problem] The present invention addresses the problem of providing: a topical composition containing a yeast extract comprising yeast-derived dietary fiber; and a method for improving skin quality and a hair care method that use this topical composition.

[Solution] The moisture content and melanin content of the skin can be improved, and the adhesion of microscopic substances such as pollen and PM2.5 to the skin can be suppressed, by applying onto the skin a yeast extract comprising yeast-derived dietary fiber with a prescribed particle size distribution. Moreover, by use on the hair, hair end evenness can be imparted and the combability can be improved, and in addition the cuticle can be protected from, e.g., ultraviolet radiation.

## Description

### Technical Field

The present invention relates to a topical composition produced by using a yeast extract containing yeast-derived dietary fiber.

### Background Art

Yeast extracts are used in various ways such as seasoning. In addition to the use for seasoning, use for improving skin moisturization (Patent Literature 1) as an oral product is also known.

In general cosmetics, in recent years, not a synthetic product but a naturally derived product such as yeast is required from the viewpoint of safety. For example, as a topical preparation for skin, yeast derived from double cherry blossom (Patent Literature 2) and a sakekasu extract (Patent Literature 3) are known.

Yeast contains a large amount of dietary fiber containing glucan and mannan constituting the cell wall as main components, and those containing dietary fiber obtained from various raw materials as active ingredients are known. For example, a method of using a yeast cell wall fraction as a skin condition improving composition (Patent Literature 4), a cosmetic using yeast cell wall-derived β-glucan (Patent Literature 5), and the like are known.

On the other hand, when a yeast extract obtained by extracting dietary fiber from yeast is used as a topical preparation such as a cosmetic, a yeast extract that exhibits a preferable function by forming a thin film is not known.

### Citation List

### Patent Literature

Patent Literature 1: JP 2019-216617 A
Patent Literature 2: JP 2020-138951 A
Patent Literature 3: JP 2004-346045 A
Patent Literature 4: JP 2007-45714 A
Patent Literature 5: JP 2005-15348 A

### Summary of Invention

### Technical Problem

An object of the present invention is to provide a topical composition containing a yeast extract containing yeast-derived dietary fiber, and a method for improving skin quality and a hair care method using the topical composition.

### Solution to Problem

As a result of intensive studies, the present inventors have found that a yeast extract containing yeast-derived dietary fiber has a thin film-forming ability, and have completed the present invention. That is, the present invention relates to the following first to fifteenth aspects.

A first aspect is a topical composition for skin, containing a yeast extract as an active ingredient, wherein the yeast extract in a powder state has a volume-based particle diameter of 5 to 500 µm.

A second aspect is the topical composition according to the first aspect, wherein the yeast extract forms a thin film by drying a 1 wt% aqueous solution.

A third aspect is the topical composition according to the first or second aspect, wherein the yeast extract contains yeast-derived dietary fiber in an amount of 5 parts by weight or more in terms of solid content relative to the yeast extract.

A fourth aspect is the topical composition according to the first or second aspect, further containing polyols.

A fifth aspect is the topical composition according to the third aspect, further containing polyols.

A sixth aspect is a method for producing the topical composition according to the fourth aspect, including the steps of: dispersing a yeast extract containing dietary fiber in a polyol; and dispersing the yeast extract dispersed in the polyol in an aqueous solvent.

A seventh aspect is a method for producing the topical composition according to the fifth aspect, including the steps of: dispersing a yeast extract containing dietary fiber in a polyol; and dispersing the yeast extract dispersed in the polyol in an aqueous solvent.

An eighth aspect is a method for improving skin quality, including applying the topical composition according to the first or second aspect to skin.

A ninth aspect is a method for improving skin quality, including applying the topical composition according to the third aspect to skin.

A tenth aspect is a method for improving skin quality, including applying the topical composition according to the fourth aspect to skin.

An eleventh aspect is a method for improving skin quality, including applying the topical composition according to the fifth aspect to skin.

A twelfth aspect is a hair care method including applying the topical composition according to the first or second aspect to hair.

A thirteenth aspect is a hair care method including applying the topical composition according to the third aspect to hair.

A fourteenth aspect is a hair care method including applying the topical composition according to the fourth aspect to hair.

A fifteenth aspect is a hair care method including applying the topical composition according to the fifth aspect to hair.

### Advantageous Effects of Invention

Since the extract containing yeast-derived dietary fiber of the present invention can be extracted from yeast known as food, it can be produced relatively easily and inexpensively, and can be used safely. Since the yeast extract can form a thin film by preparing an aqueous solution of the yeast extract and then drying the aqueous solution, the yeast extract can exhibit a barrier function by forming a thin film of the yeast extract on the body surface. In addition, by using the topical composition containing the yeast extract, the skin quality can be improved. Thus, an increase in the stratum corneum water content, a decrease in the transepidermal water loss, an improvement in the texture, a decrease in the melanin content, and an improvement in the color tone can be achieved. In addition, when the topical composition is used for hair, cuticle can be protected, gloss and manageability can be imparted to hair, and a moisturizing effect can be imparted to the hair to suppress dryness.

### Brief Description of Drawings

Fig. 1 shows comparison of the hygroscopicity between yeast extract and other materials.
Fig. 2 shows changes in appearance of yeast extract and other materials before and after moisture absorption.
Fig. 3 shows comparison of the water retention rate between yeast extract and other materials.
Fig. 4 shows the thin film-forming ability of yeast extract.
Fig. 5 shows comparison of substance permeability between yeast extract and other materials.
Fig. 6 shows concentration dependence of change in transepidermal water loss by yeast extract.
Fig. 7 shows long-lasting moisturizing effect test with yeast extract.
Fig. 8 shows an effect of imparting gloss to damaged hair by yeast extract.
Fig. 9 shows a moisturizing effect on damaged hair by yeast extract.
Fig. 10 shows an effect of improving manageability of damaged hair by yeast extract.
Fig. 11 shows an effect of improving hair manageability by yeast extract.
Fig. 12 shows an effect of protecting cuticle from ultraviolet rays by yeast extract.

### Description of Embodiments

Any yeast can be used for producing the extract containing dietary fiber of the present invention as long as it is a commonly-used yeast. Specifically, baker's yeast, brewer's yeast (*Saccharomyces cerevisiae*)*,* and torula yeast (also referred to as (*Cyberlindnera jadinii*); *Candida utilis*) are exemplified. Among them, torula yeast is particularly desirable.

As the yeast cells, yeast cells obtained by culturing yeast may be used as they are, or yeast cells rich in cell wall fractions generated in general yeast extract production may be used. In addition, those obtained by drying these yeast cells can also be used. Particularly desirable are yeast cells rich in cell wall fraction, which are generated when torula yeast cells are extracted with a water-soluble solvent.

A yeast extract containing yeast-derived dietary fiber can be obtained by washing the yeast cells with alkaline ion water and allowing glucanase to act on the yeast cells. The alkaline ion water is water containing a large amount of hydroxide ions, and is obtained by electrolyzing water. The alkaline ion water is adjusted to pH 8 to 12, and preferably pH 9 to 11. The pH may be adjusted before washing or after suspension of the cells. The temperature is adjusted to 50 to 90°C, and desirably 60 to 80°C. Similarly to the pH adjustment, the temperature may be adjusted before or after suspension of the cells. In this way, the yeast cells are washed with alkaline ion water adjusted as necessary. The washing method may be any method that can be selected by those skilled in the art. For example, there is a method in which yeast cells are suspended in alkaline ion water so as to be about 10 wt%, and after the suspension, the yeast cells are recovered by centrifugation and then washed. The number of times of washing is also arbitrary, and washing may be performed a plurality of times.

After washing, enzyme treatment is performed. As the glucanase used for the enzyme treatment, a glucanase derived from a microorganism such as *Streptomyces* or *Taloromyces* can be used. Those obtained by causing these enzymes to be expressed in an appropriate host cell may be used, or a generally available glucanase preparation may be used. The conditions for the enzyme treatment can be appropriately set with reference to the optimum conditions of the enzyme to be used and the conditions under which the activity of coexisting contaminating enzymes is low. For example, in the case of a glucanase preparation "DENAZYME GEL", the yeast extract is adjusted to pH 4 to 7, preferably pH 5 to 6, and the enzyme is added to the yeast extract at 25 to 75°C, preferably 30 to 60°C, in an amount of 0.05 to 2%, preferably 0.1 to 1% of the weight of the yeast cells, and reacted for 1 to 30 hours, preferably 10 to 15 hours.

After the enzymatic reaction, the solid contents are removed. Any removal method can be adopted, but a method of separating by centrifugation, a method of removing with a filtration membrane, or the like can be adopted. A method of removing by centrifugation is preferable. The yeast extract of the present invention thus obtained contains dietary fiber. The yeast extract of the present invention contains 5 wt% or more, desirably 15 wt% or more, and further desirably 50 wt% or more of dietary fiber.

The obtained yeast extract can be prepared as a powder by a general drying process such as spray drying as necessary.

The yeast extract of the present invention prepared as a powder by the drying process has a volume-based particle diameter within a range of 5 to 500 µm, preferably 8 to 400 µm, and more preferably 10 to 350 µm. The volume-based particle diameter is measured by a laser diffraction scattering method (wet process) after suspending the yeast extract in isopropanol and sonicating the yeast extract. Furthermore, the yeast extract preferably has a volume average diameter within a range of 50 to 200 µm.

The yeast extract containing the yeast-derived dietary fiber of the present invention can be obtained by the preceding method. The yeast extract thus obtained is excellent in the ability to adsorb and retain moisture, and can retain moisture even under a low humidity condition.

The yeast extract containing yeast-derived dietary fiber of the present invention has a property of forming a thin film that can limit the permeation of substances. Particularly, when an aqueous solution of about 1 wt% of the yeast extract is applied and spread, and then dried on a substrate, the yeast extract of the present invention has a property of forming a thin film having a fine mesh structure. When the surface of the thin film prepared from a 1 wt% aqueous solution of the yeast extract is observed with an electron microscope, a fine mesh structure having pores with a major axis of about 3 to 15 µm can be confirmed. A formed thin film having no pores, as a matter of course, and also a formed thin film having pores has a property of limiting permeation of substances depending on the size of the pores. For example, it is possible to limit permeation of microscopic air pollutants such as pollen and PM2.5 and to suppress adhesion of these substances to skin. The concentration of the yeast extract used as a topical composition may be a concentration at which pores are not formed on the surface of the thin film. It is also possible to impart flexibility to the thin film by adding a polyol described later.

The content of the topical composition of the present invention can be set such that the above described yeast extract can be appropriately adjusted by those skilled in the art of topical composition production. The yeast extract may be used as it is as a topical composition, but is desirably contained in the topical composition in an amount of 0.01 to 15 wt%, preferably 0.05 to 12 wt%, and more preferably 0.1 to 10 wt%. Furthermore, the topical composition of the present invention may be mixed with a material of another topical preparation to prepare a topical composition.

The topical composition of the present invention may further contain a polyol. The polyol in the present invention refers to an aliphatic compound having two or more hydroxy groups, or a carbohydrate in which at least a part of carbonyl groups in the molecule is reduced into a hydroxy group. As the polyol used in the present invention, generally available polyols can be used, and examples thereof include glycerol, erythritol, arabitol, xylitol, mannitol, maltitol, 1,3-butylene glycol, propylene glycol, 3-methyl-1,3-butanediol, 1,3-propanediol, 2-methyl-1,3-propanediol, trimethylolpropane, pentaerythritol, sorbitol, pentylene glycol, hexylene glycol, 1,2-hexanediol, caprylyl glycol, diglycerin, polyglycerin, diethylene glycol, polyethylene glycol, dipropylene glycol, polypropylene glycol, and a hydrogenated starch hydrolysate. The amount of the polyol is preferably 0.05 to 20 parts by weight, more preferably 0.1 to 18 parts by weight, and still more preferably 0.2 to 15 parts by weight, based on 1 part by weight of the yeast extract.

In addition, the material that can be used for the topical composition of the present invention is not limited. The topical composition of the present invention may contain, for example, various active ingredients such as oils, alcohols, ethers, surfactants, sequestering agents, pearl agents, amino acids, organic amines, water-soluble polymers, polymer emulsions, pH adjusters, vitamins, antioxidants, preservatives, water-soluble polymers, fragrances, ultraviolet absorbers, and ultraviolet ray blockers. The form of the topical composition is not limited as long as the topical composition contains the yeast extract of the present invention and can be applied to skin and hair. The topical composition can be prepared and used in the form of a solution, a dispersion, a paste, a gel, or a sheet in accordance with an ordinary method using a commonly-used aqueous solvent. The mixing amount is also not limited, and can be a content that can be appropriately adjusted by those skilled in the art of topical composition production.

When the polyol is used for the topical composition, the polyol may be mixed simultaneously with the yeast extract or other materials, or may be added and mixed separately. In addition, yeast extract dispersed in a polyol in advance may be dispersed in an aqueous solvent usable for a topical composition to prepare a topical composition.

The skin quality can be improved using the topical composition of the present invention obtained by the preceding method. Here, the improvement of the skin quality in the present invention refers to improvement of the water retention capacity, improvement of the skin elasticity, improvement of the texture, and improvement of the skin tone. The water retention capacity can be evaluated from the water loss and the skin water content. In addition, the skin elasticity can be evaluated by the maximum suction height measured with a cutometer. In addition, the skin texture can be evaluated by an ordinary method such as a replica method, and the improvement of the color tone can be evaluated by ROBOSKIN ANALYZER or the like.

In addition, the topical composition of the present invention obtained by the preceding method can be used for the purpose of hair care. Hair care in the present invention refers to protection of cuticle, moisturization of hair, and suppression of dryness, as well as imparting gloss and manageability to hair and improving smoothness of combing. Hair care can be evaluated by any of appearance such as hair manageability and gloss, smoothness of combing, and water loss when the topical composition of the present invention is applied to hair by a method such as application or spraying and dried. In addition, the topical composition of the present invention can also be used as a hairdressing preparation.

### Examples

Hereinafter, the present invention will be further described in detail with reference to Examples, however the present invention is not limited to these Examples.

### (Production of yeast extract containing yeast-derived dietary fiber)

To 10 g of torula yeast cells, 90 g of alkaline ion water was added to adjust the concentration to 10%, and the cells were heated to 70°C. The pH at that time was 9.5. Thereafter, centrifugation treatment was performed, and the cells were washed. Then, 200 g of pure water was added to suspend the washed cells in the pure water, and then the cells were washed again by centrifugation. Then, 90 g of water was added to the washed cells, and 0.05 g of DENAZYME GEL was added at 60°C and pH 6.5. The mixture was reacted for 3 hours with stirring after addition, and after the reaction, each was centrifuged to obtain a supernatant. The supernatant was recovered as an extract containing dietary fiber. After the recovering, the extract was spray-dried to obtain 2.5 g of a dried product. The obtained dried product was used as sample 1. The dietary fiber content in sample 1 was 78 wt% as measured by an enzyme-HPLC method. In addition, the particle size distribution of sample 1 dispersed in isopropanol was measured by a laser diffraction scattering method (Microtrac MT3000EX, wet process), and the volume average diameter was 81.58 µm.

### (Hygroscopicity test)

Sample 1 as a sample to be subjected to the hygroscopicity test, and indigestible dextrin, inulin, isomaltodextrin, glucan derived from barley and guar gum (all samples for comparison are commercially available as reagents) as comparison targets were each weighed in an amount of about 3 g in a weighing dish (n = 5). Each sample whose weight was measured was placed in a thermo-hygrostat, held at a relative humidity of 75% and 40°C, and taken out at the time when 1, 2, 4, 6, and 8 hours had elapsed, and the weight of each sample was measured. The moisture absorption rate was calculated from the amount of increase in the weight of the sample before and after the sample was placed in the thermo-hygrostat.

### (Results of moisture absorption test)

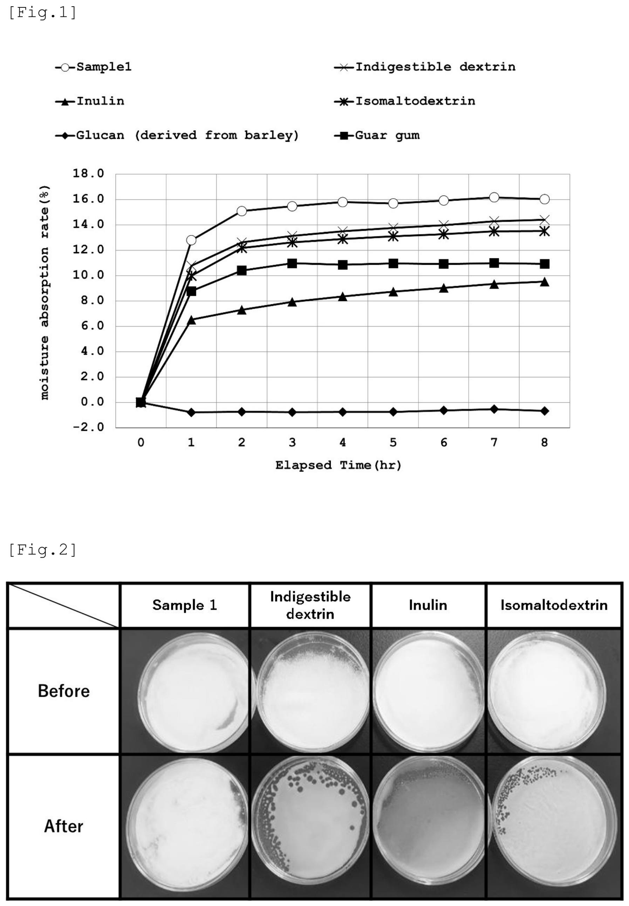

The results are shown in Fig. 1. As a result, sample 1 showed the highest moisture absorption rate. On the other hand, glucan derived from barley showed the lowest moisture absorption rate. Furthermore, sample 1, the indigestible dextrin, inulin and isomaltodextrin were placed in a thermo-hygrostat, and the appearance thereof was confirmed after a lapse of 18 hours. As a result, the samples for comparison showed deliquescence, whereas sample 1 did not show deliquescence, and showed properties as a powder (Fig. 2). From the above results, it was revealed that the yeast extract of the present invention had characteristic physical properties of not exhibiting deliquescence while having high hygroscopicity.

### (Water retention rate test)

Sample 1 was used as a sample to be subjected to a water retention rate test. In addition, a culture solution of black yeast (Accession No. FERMP-20869) was dried, and the resulting culture containing glucan (β-1,3-1,6-glucan 0.8 wt%) was used as a sample for comparison. Furthermore, a change in water retention rate was examined using γ-polyglutamic acid (FUJIFILM Wako Pure Chemical Corporation) commercially available as a reagent as another sample for comparison. Each sample was placed in a weighing dish in an amount of about 5 g, and the weight at a humidity of 0% was measured. Each weighed sample was held in a high humidity condition (relative humidity 65%) for 24 hours in a high temperature and humidity apparatus, and then the weight was measured. Subsequently, the sample was held under a low humidity condition (relative humidity 35%) for 48 hours, then the weight was measured, the rate of change in weight was calculated, and the water retention rate was evaluated.

### (Test results of water retention rate)

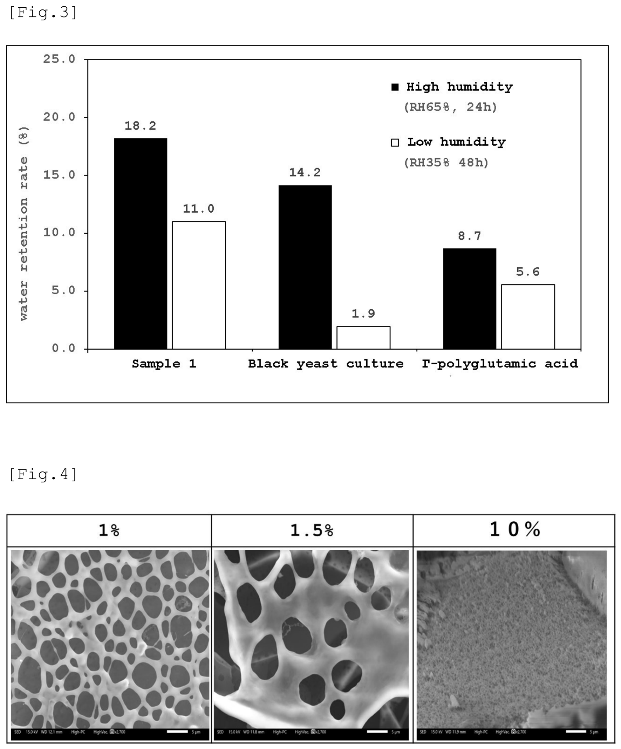

The results are shown in Fig. 3. Sample 1 showed a high moisture absorption rate under a high humidity condition as compared with the black yeast culture and γ-polyglutamic acid. Furthermore, after the treatment under a low humidity condition, sample 1 showed a higher water retention rate as compared with the black yeast culture. From the above results, it was revealed that the yeast extract (sample 1) of the present invention had a high water retention rate even under a low humidity condition.

### (Thin film-forming ability)

A thin film obtained by applying and spreading each of 1 wt%, 1.5 wt%, and 10 wt% aqueous solutions of sample 1 onto a substrate with a thickness of about 2 mL/cm^2, and drying each solution was observed by an electron microscope (JCM-7000; JEOL Ltd.) (Fig. 4). As a result, it was revealed that the thin film prepared by drying the 1.5 wt% aqueous solution had a fine mesh structure having pores with a major axis of about 12 µm at a maximum. The thin film prepared by drying the 1.5 wt% aqueous solution had slightly larger pores as compared with the thin film prepared from the 1.0 wt% aqueous solution. On the other hand, with the 10 wt% aqueous solution, a fine mesh structure was not formed, but a thin film could be obtained.

### (Confirmation test of barrier performance)

The substance permeation limiting ability of the thin film derived from the yeast extract was examined. The 1 wt% aqueous solution of sample 1 was prepared and used for the test. In addition, a 1% aqueous solution of the above-described black yeast culture was prepared and used as a comparison target. To half of an artificial skin model (BIOSKIN plate P001-001 #W; Beaulax Co., Ltd), 10 µL of each aqueous solution was applied, and air-dried for 1 hour. Thereafter, 10 µL of an activated carbon suspension prepared to contain 10 wt% of activated carbon having a particle size of 3 µm was applied to the entire artificial skin model. After 1 hour, water was poured onto the artificial skin model to remove the sample, and an image of each artificial skin model was captured for image analysis. The data of the captured image was converted into a binary image of white and black using image processing software. Then, the area of black in the area coated with the activated carbon suspension was calculated to quantify the amount of the remaining activated carbon. A lower numerical value indicates that the amount of activated carbon attached is small and the substance permeation limiting ability is high.

### (Test results of barrier performance)

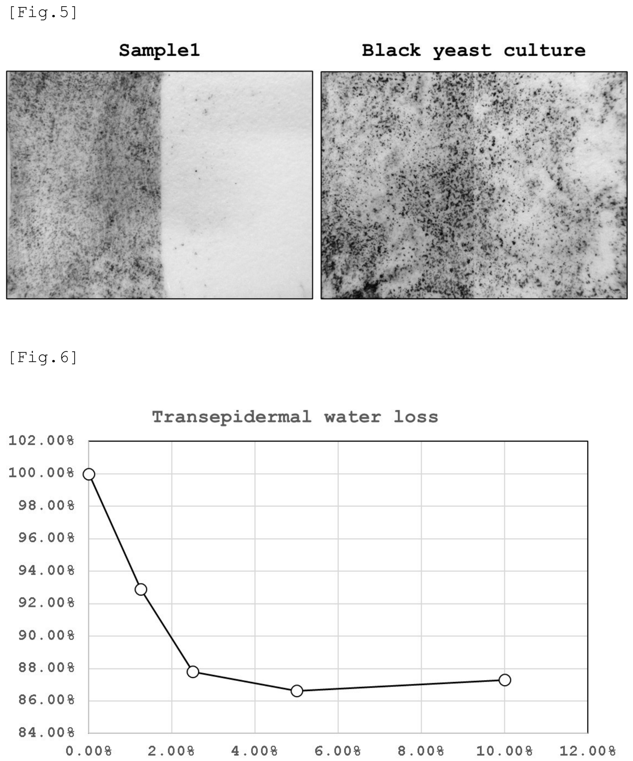

The captured image is shown in Fig. 5. In each of the test sections, the left half is the artificial skin model of a control section (only application of activated carbon suspension). From the results of Fig. 5, in sample 1, the adhesion of activated carbon particles was suppressed as compared with the site where no sample was applied. On the other hand, in the test section of the black yeast culture, the adhesion of activated carbon particles was observed. Furthermore, the amount of activated carbon remaining in the artificial skin model was calculated as the area of a black portion (remaining activated carbon particles) occupying a certain area of the image in image processing software, and is shown in the table (Table 1). The numerical values of the image analysis also revealed that in sample 1, the residual amount of activated carbon was smaller as compared with the black yeast culture, and thus the substance permeation limiting ability was high.

**[Table 1]**

| (%Area) | Sample 1 | black yeast culture |
|---|---|---|
| Control section | 55.974 | 48.109 |
| applied section | 0.141 | 19.381 |

### (Concentration dependence test)

Sample 1 was dissolved in water to prepare 1.25%, 2.5%, 5% and 10% (all in wt%) aqueous solutions. Six test sections of about 1.5 cm square were provided side by side at intervals on the inner side of the forearm of three healthy men who agreed with the test. Among the six test sections, two test sections at both ends were defined as a test section where sample 1 was not applied, and the remaining four test sections were defined as a test section where a solution of sample 1 at each concentration was applied. The test method was performed according to the following procedure. The inner side of the forearm of the subject was thoroughly washed with soap and then lightly wiped with a paper towel, and the subject waited for 15 minutes or more. Six test sites were marked, and 20 µL of each of the solutions of sample 1 at each concentration was taken, applied to each test section, and blended into the skin with fingers and then dried. After a further 10 minutes, the transepidermal water loss (TEWL) at each test site was measured with Tewameter TM HEX (Courage+ Khazaka). The TEWL was measured at least twice in each test section, and the third measurement was performed only when the values were largely deviated. The values of the four test sections were calculated as relative values with the measured value of the non-applied section taken as 100%, and a significant difference test was performed with a t-test (both sides). A smaller value of TEWL indicates a higher moisturizing effect.

### (Results of moisture retention test)

The results are shown in Fig. 6. From this result, it was confirmed that the TEWL tended to decrease even in the test section to which 1.25% of sample 1 was applied. Furthermore, it was revealed that the TEWL was significantly decreased in the test sections to which 2.5% or more of sample 1 was applied. From the above results, it was revealed that sample 1 was effective in moisturizing at 1.25% or more when applied to the human skin. Since the yeast extract of the present application forms a thin film at 1% or more, it is considered that the thin film formed on the skin contributed to the decrease in the water loss.

### (Long-lasting moisturizing effect test)

In order to confirm the long-lasting moisturizing effect, a test gel was prepared according to the composition in Table 2. The test gel was prepared by dispersing sample 1 in a mixture of butylene glycol, pentylene glycol, and glycerin, and then dispersing the resulting dispersion in a mixture obtained by mixing other raw materials in advance. A test section of 2 cm square was provided on the inner side of the forearm of ten healthy women who agreed with the test, and 20 µL of the prepared test gel was applied and spread with fingers. The TEWL was measured immediately after application, after 30 minutes, and every 1 hour after 1 hour to 6 hours, and an average value was calculated. A smaller value of TEWL indicates a higher moisturizing effect.

**[Table 2]**

| Component | Proportion(%) |
|---|---|
| 1% Hydroxyethyl cellulose solution | 6.00 |
| 2% (Acrylates/alkyl (C10-30) acrylate) crosspolymer aqueous solution | 20.00 |
| Butylene glycol | 10.00 |
| Sample 1 | 1.50 |
| Pentylene glycol | 4.00 |
| Glycerin | 4.00 |
| Sodium hydroxide | 0.137 |
| Phenoxyethanol | 0.30 |
| purified water | Up to 100% |
| total | 100.00 |

### (Results of long-lasting moisturizing effect test)

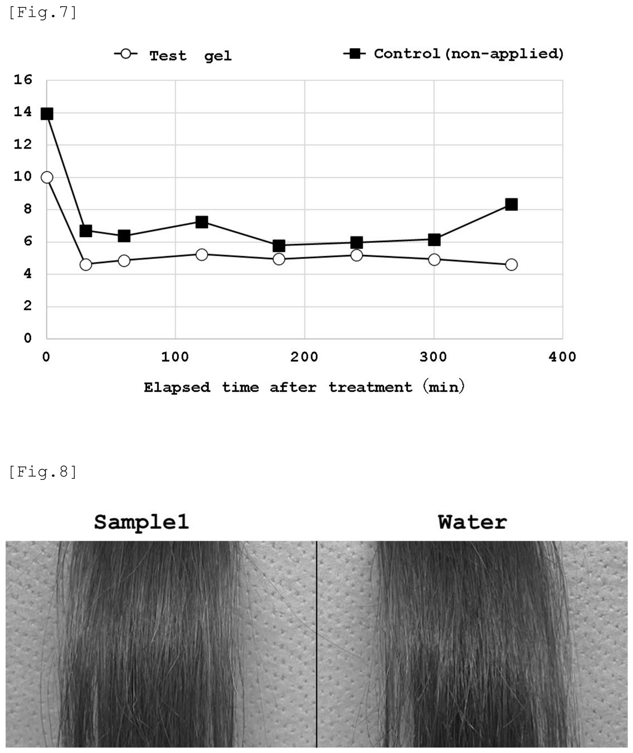

The results of the long-lasting moisturizing effect test are shown in Fig. 7. Comparison with the value of TEWL in the non-applied section showed that the value of TEWL was low in any case and the water loss via the skin was suppressed to be low, and the effect was maintained at least until 6 hours after application.

### (Long-term continuous use test)

In order to confirm the long-lasting moisturizing effect, a test cream was prepared according to the composition in Table 3. Twenty healthy women who agreed with the test were asked to apply about 0.8 g of a test cream to the face after using a lotion after makeup removal and face washing two times in the morning and night for 28 days. The evaluation items were skin water content, elasticity, melanin content, skin texture, and skin tone. The measurement was performed before use of the test cream and after 28 days from use of the test cream. The measurement was performed for each item after acclimatization in a constant temperature and constant humidity environment (room temperature 22±2°C, humidity 50±10%) for 20 minutes. The skin water content (measurement site: right cheek) was measured at least twice using a corneometer (Courage+ Khazaka), and the third measurement was performed when the values were largely deviated, and the average value was calculated. A higher measured value of the skin water content indicates a higher skin water content. The elasticity (right cheek measurement site:) was evaluated by measuring the maximum suction height with a cutometer (Courage+ Khazaka) having a probe measurement opening of 2 mm. A higher measured value of elasticity indicates better elasticity. The skin texture (measurement site: right cheek) was measured by a replica method and evaluated by a skin texture volume ratio. The melanin content (measurement site: right cheek) was evaluated using a Mexameter MX18. The skin tone (front side) was evaluated by ROBOSKIN ANALYZER CS50.

**[Table 3]**

| Component | Proportion(%) |
|---|---|
| Blend of Behenyl Alcohol / Polyglyceryl-10 Pentastearate / Sodium Stearoyl Lactylate | 2 |
| sqalane | 5 |
| Blend of Lecithin / lysophosphatidic acid / lysophosphatidic acid | 0.1 |
| Butylene glycol | 10 |
| Sample 1 | 1.5 |
| Pentylene glycol | 4 |
| Glycerin | 4 |
| | 5 |
| 2% (Acrylates/alkyl (C10-30) acrylate) crosspolymer aqueous solution | 20 |
| 10%-sodium hydroxide | 1.367 |
| Phenoxyethanol | 0.5 |
| purified water | Up to 100% |
| total | 100 |

### (Results of long-term continuous use test)

First, the skin water content was 47.8±7.5 before application of the test cream, whereas the skin water content was 58.5±9.6 4 weeks after application. This revealed that the skin water content significantly increased (p < 0.001). Next, the elasticity was 0.238±0.061 mm before application of the test cream, whereas the elasticity was 0.316±0.063 mm 4 weeks after application. This showed that the elasticity significantly increased (p < 0.001). Subsequently, the skin texture volume ratio was 23.3±13.0 µm^3/mm^2/100 before application of the test cream, whereas the skin texture volume ratio was 30.4±10.5 µm^3/mm^2/100 4 weeks after application. This showed that the skin texture was significantly improved (p < 0.01). In addition, the melanin content of the skin was 133.7±21.8 before application of the test cream, whereas the melanin content was 125.4±16.8 4 weeks after application, and the melanin content significantly decreased (p < 0.05). Finally, the skin tone (brightness) was 73.9±2.2 before application of the test cream, whereas the skin tone was 74.4±2.0 4 weeks after application, and a significant difference was observed (p < 0.05). From the above results, improvement was observed in all items.

### (Preparation of damaged hair)

In order to confirm the application effect to the hair, human black hair subjected to a damage treatment is used. The damaged hair was prepared in the following procedure. Human black hair (Beaulax Co., Ltd.) was immersed in a solvent prepared by mixing equal amounts of acetone, isopropyl alcohol, ethanol, and water, respectively, for 24 hours to perform a degreasing treatment. Subsequently, the degreased hair was immersed in a 1 wt% SDS aqueous solution at 40°C for 30 minutes. Furthermore, the hair was subjected to a bleaching treatment with a commercially available hair bleaching agent for 20 minutes to prepare damaged hair.

### (Confirmation of gloss and measurement of secondary transpiration water content)

Confirmation of the moisturizing effect using damaged hair was performed by the following procedure. The damaged hair was shampooed and then dried with a dryer, and 1 mL of water or an aqueous solution of sample 1 (1 wt%) was sprayed thereon, then lightly dried with the dryer, and allowed to stand at room temperature for about 1 hour. Thereafter, the appearance was observed to confirm gloss (Fig. 8). As a result, the damaged hair sprayed with the aqueous solution of sample 1 was found to be well-behaved and glossy, whereas the damaged hair sprayed with water was found to be dry and not glossy.

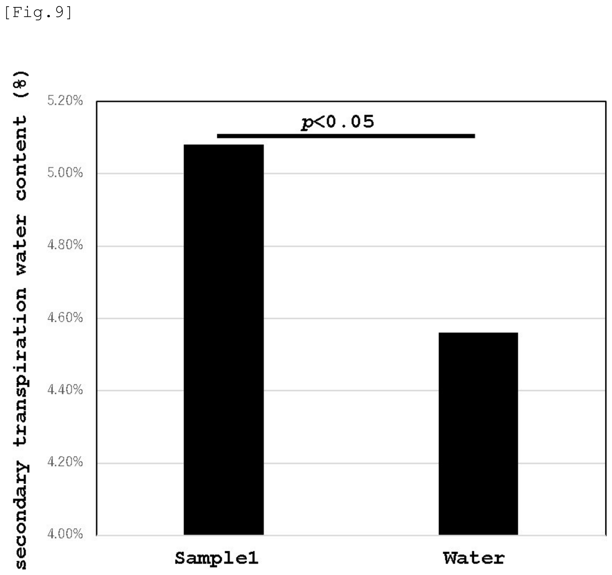

Furthermore, about 0.3 g of each damaged hair was cut so as to include the hair ends to hair roots, treated at 65°C for 30 minutes, and then weighed (weight A). Subsequently, the damaged hair was treated at 180°C for 30 minutes, and the weight (weight B) was measured. The secondary transpiration water content of each damaged hair was calculated from an equation "secondary transpiration water content (%) = 100* (weight A- weight B)/weight B" using the numerical values of the weight A and the weight B of each obtained damaged hair. The secondary transpiration water content of each damaged hair was subjected to a t-test (n = 3, both sides). The results are shown in Fig. 9. The damaged hair sprayed with the aqueous solution of sample 1 showed a significantly higher secondary transpiration water content as compared with the damaged hair sprayed with water (p < 0.05). From this, it was revealed that the composition of the present invention could retain moisture contained in hair.

### (Confirmation of effect of improving hair manageability)

The damaged hair was shampooed and then dried with a towel, 1 mL of water or an aqueous solution of sample 1 was sprayed on the entire hair, and then dried with a dryer, and acclimatized at a humidity of about 60% and 24.5°C for 20 minutes, and then the damaged hair was observed.

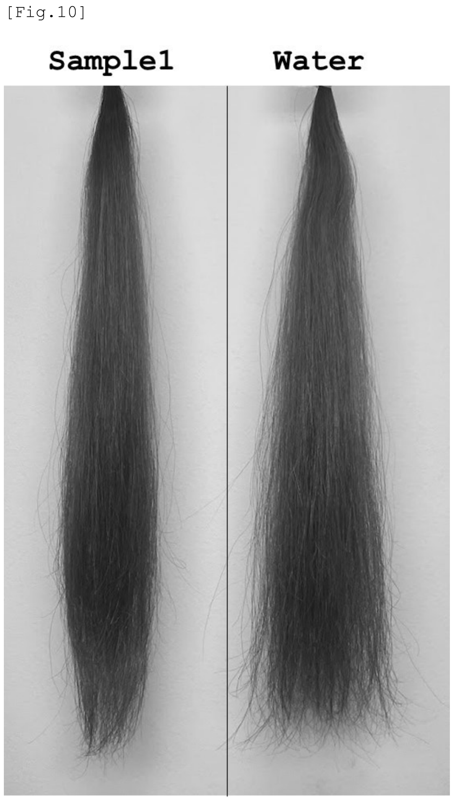

The results are shown in Fig. 10. The damaged hair sprayed with the aqueous solution of sample 1 was suppressed from spreading, and hair ends were well-behaved. On the other hand, the damaged hair sprayed with water was well-behaved immediately after drying with a dryer, but spread over time.

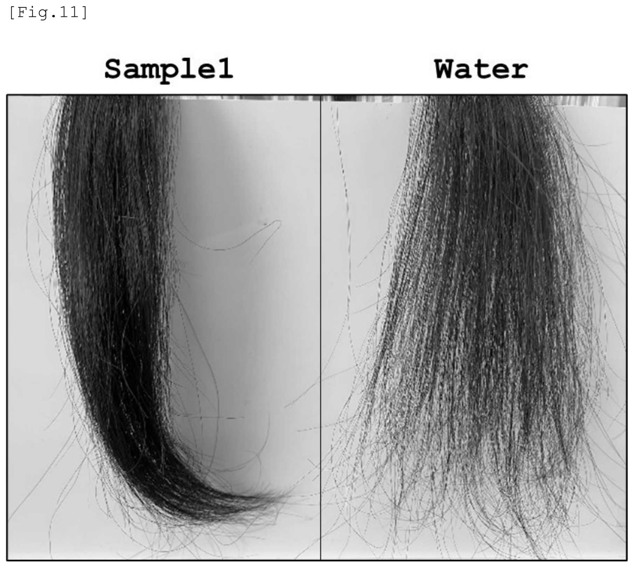

The same treatment was also performed on the human black hair that had not been subjected to the damage treatment, and hair manageability was observed (Fig. 11). As a result, in the human black hair sprayed with sample 1, dryness was suppressed, hair ends were well-behaved, and smoothness of combing was improved. On the other hand, in the human black hair sprayed with water, hair ends were not well-behaved.

From the above results, it was shown that the composition of the present invention could improve hair manageability.

### (Confirmation of effect of protecting cuticle of hair)

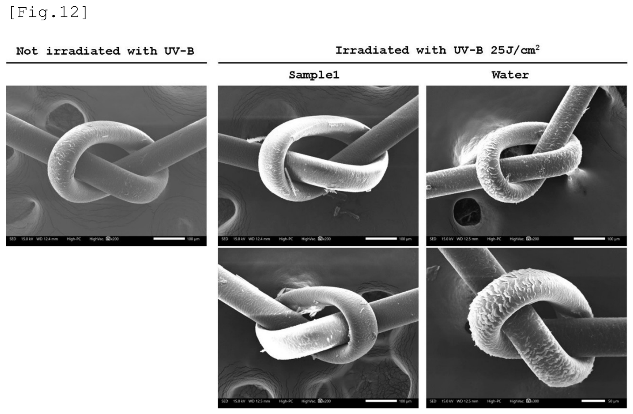

After irradiating the hair with ultraviolet rays, the cuticle was observed to confirm the effect of protecting cuticle of the hair by the composition of the present invention. The test method was performed according to the following procedure. Human black hair (Beaulax Co., Ltd.) was shampooed twice. An operation of applying 1 mL of water or an aqueous solution of sample 1 (0.5 wt%) to the hair and then drying with a dryer was repeated 5 times. Thereafter, water was applied to the hair, and the hair was irradiated using an ultraviolet lamp (manufactured by Sankyo Electronics Co., Ltd.; GL20SE) at a distance of 15 cm with an intensity of UV-B25 J/cm^2 for 5 hours. After ultraviolet irradiation, the cuticle was observed using an electron microscope (Fig. 12). As a result, the human black hair sprayed with the aqueous solution of sample 1 had the same appearance as the hair not irradiated with ultraviolet rays. On the other hand, the cuticle was peeled off from the human black hair sprayed with water. Furthermore, when the human hair sprayed with sample 1 is compared with the human hair not irradiated with UV-B, it had the same appearance as the human hair not irradiated with UV-B. From this, it was revealed that the composition of the present invention could suppress the cuticle from being peeled off by ultraviolet irradiation and protect the cuticle.

From the above results, the topical composition containing the yeast extract of the present invention has a thin film-forming ability and can suppress the transepidermal water loss, and thus can be used as a topical composition for skin. The topical composition of the present invention can also be used for moisturization and skin firmness improvement by being applied to the skin. Furthermore, by using the topical composition containing the yeast extract of the present invention for hair, cuticle can be protected, gloss and manageability can be imparted to hair, and smoothness of combing can be improved, so that the topical composition can be used for hair care.

## Claims

1. A topical composition for skin, comprising a yeast extract as an active ingredient, wherein the yeast extract in a powder state has a volume-based particle diameter of 5 to 500 µm.

2. The topical composition according to claim 1, wherein the yeast extract forms a thin film by drying a 1 wt% aqueous solution.

3. The topical composition according to claim 1 or 2, wherein the yeast extract contains yeast-derived dietary fiber in an amount of 5 parts by weight or more in terms of solid content relative to the yeast extract.

4. The topical composition according to claim 1 or 2, further comprising polyols.

5. The topical composition according to claim 3, further comprising polyols.

6. A method for producing the topical composition according to claim 4, comprising the steps of: dispersing a yeast extract containing dietary fiber in a polyol; and dispersing the yeast extract dispersed in the polyol in an aqueous solvent.

7. A method for producing the topical composition according to claim 5, comprising the steps of: dispersing a yeast extract containing dietary fiber in a polyol; and dispersing the yeast extract dispersed in the polyol in an aqueous solvent.

8. A method for improving skin quality, comprising applying the topical composition according to claim 1 or 2 to skin.

9. A method for improving skin quality, comprising applying the topical composition according to claim 3 to skin.

10. A method for improving skin quality, comprising applying the topical composition according to claim 4 to skin.

11. A method for improving skin quality, comprising applying the topical composition according to claim 5 to skin.

12. A hair care method comprising applying the topical composition according to claim 1 or 2 to hair.

13. A hair care method comprising applying the topical composition according to claim 3 to hair.

14. A hair care method comprising applying the topical composition according to claim 4 to hair.

15. A hair care method comprising applying the topical composition according to claim 5 to hair.
